(19) Europäisches Patentamt
European Patent Office
Office européen des brevets

(11) Veröffentlichungsnummer: 0 176 741
B1

(12) # EUROPÄISCHE PATENTSCHRIFT

(45) Veröffentlichungstag der Patentschrift: **26.10.88**

(51) Int. Cl.[4]: **C 07 D 213/79,** C 07 D 213/80, A 61 K 31/44

(21) Anmeldenummer: **85110498.4**

(22) Anmeldetag: **21.08.85**

(54) **Ester der Pyridin-2,4 - und -2,5-dicarbonsäure als Arzneimittel zur Inhibierung der Prolin- und Lysinhydroxylase.**

(30) Priorität: **31.08.84 DE 3432094**

(43) Veröffentlichungstag der Anmeldung: **09.04.86 Patentblatt 86/15**

(45) Bekanntmachung des Hinweises auf die Patenterteilung: **26.10.88 Patentblatt 88/43**

(84) Benannte Vertragsstaaten: **AT BE CH DE FR GB IT LI LU NL SE**

(56) Entgegenhaltungen:
**FR - A - 2 123 141**
**FR - M - 3 795**
**US - A - 2 852 519**

**CHEMICAL ABSTRACTS, Band 49, Nr. 14, 25. Juli 1955, Columbus, Ohio, USA J. PLIML "Synthesis analogs of curare alkaloids"**

(73) Patentinhaber: **HOECHST AKTIENGESELLSCHAFT, Postfach 80 03 20, D-6230 Frankfurt am Main 80 (DE)**

(72) Erfinder: **Günzler, Volkmar, Wormser Strasse 66, D-6501 Bodenheim (DE)**
Erfinder: **Hanauske-Abel, Hartmut, Dr., Schlossstrasse 35, D-6501 Dexheim (DE)**
Erfinder: **Mohr, Jürgen, Dr., Birkenweg 18, D-6094 Bischofsheim (DE)**
Erfinder: **Tschank, Georg, Dr., An den Mühlwegen 34, D-6500 Mainz 42 (DE)**
Erfinder: **Kivirikko, Kari, Prof. Dr., Lämsantie 14 B 1, SF-90230 Oulu (FI)**
Erfinder: **Majamaa, Kari, Dr., 340, South Miraleste 276, San Pedro California 90732 (US)**
Erfinder: **Brocks, Dietrich, Dr., Buchenstrasse 1, D-6257 Hünfelden (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft Verbindungen der Formel (I)

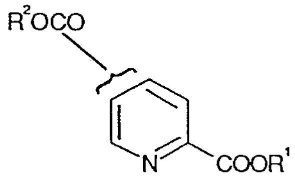

I

in der $R^1$ und $R^2$, die gleich oder verschieden sind, Alkyl mit 1 bis 6 Kohlenstoffatomen bedeuten kann, wobei die Gruppe $R^2OCO$ in 4- oder 5-Stellung des Pyridinrings gebunden ist, zur Anwendung als Arzneimittel, insbesondere zur Inhibierung der Prolin- und Lysinhydroxylase als Fibrosuppressivum und Immunsuppressivum. Die Erfindung betrifft auch Arzneimittel, die aus einer Verbindung der Formel (I) bestehen oder eine solche enthalten. Die Erfindung betrifft weiterhin die Verwendung von Verbindungen der Formel (I) zur Herstellung von Arzneimitteln für die Beeinflussung des Stoffwechsels von Kollagen und kollagenähnlichen Stoffen und der Biosynthese von $C1_q$ bzw. zur Herstellung von Arzneimitteln für die Behandlung von Störungen des Stoffwechsels solcher Moleküle sowie Verfahren zur Herstellung von Arzneimitteln zur Beeinflussung des Stoffwechsels von Kollagen und kollagenähnlichen Stoffen bzw. der Biosynthese $C1_q$.

Erfindungsgemäss werden solche Verbindungen der Formel (I) bevorzugt, in denen beide Reste $R^1$ und $R^2$ für die genannten Alkylreste stehen, vorzugsweise solche mit 1 bis 3 Kohlenstoffatomen, insbesondere für Ethyl.

Aus der EP-B 0033151 (US-A 4 457 936) ist es bekannt, dass Carbonsäuren des Hydroxyphenyl-Thiazols und seiner Derivate den Kollagenstoffwechsel beeinflussen. Diese wirken als Inhibitoren der Prolin- und Lysinhydroxylase und bewirken so eine sehr selektive Hemmung der Kollagenbiosynthese durch Beeinflussung der kollagenspezifischen Hydroxylierungsreaktionen. In deren Verlauf wird proteingebundenes Prolin oder Lysin durch die Enzyme Prolin- bzw. Lysinhydroxylase hydroxyliert. Wird diese Reaktion durch Inhibitoren unterbunden, so entsteht ein nicht-funktionsfähiges, unterhydroxyliertes Kollagenmolekül, das von der Zelle nur in geringer Menge in den extrazellulären Raum abgegeben werden kann. Das unterhydroxylierte Kollagen kann ausserdem nicht in die Kollagenmatrix eingebaut werden und wird sehr leicht proteolytisch abgebaut. Als Folge dieser Effekte verringert sich insgesamt die Menge des extrazellulär abgelagerten Kollagens.

Es ist ausserdem bekannt, dass die Inhibierung der Prolinhydroxylase durch bekannte Inhibitoren wie $\alpha,\alpha'$-Dipyridyl zu einer Hemmung der $C1_q$-Biosynthese von Makrophagen führt (W. Müller et al., FEBS Lett. 90 (1978) 218; Immunobiology 155 (1978) 47). Dadurch kommt es zu einem Ausfall des klassischen Weges der Komplementaktivierung. Inhibitoren der Prolinhydroxylase wirken daher auch als Immunsuppressiva, z.B. bei Immunkomplexkrankheiten.

Es ist bekannt, dass Prolinhydroxylase durch Pyridin-2,4- und -2,5-dicarbonsäure effektiv gehemmt wird (K. Majamaa et al., Eur. J. Biochem. 138 (1984) 239–245). Diese Verbindungen sind in der Zellkultur allerdings nur in sehr hohen Konzentrationen als Hemmstoffe wirksam (V. Günzler et al., Collagen and Related Research 3 (1983) 71). Überraschenderweise wurde nun gefunden, dass Ester dieser Verbindungen hochaktive Hemmstoffe der Kollagenbiosynthese in der Zell- und Organkultur sind.

Die Hemmwirkung der Substanzen auf die Kollagenbiosynthese kann in der Zellkultur mit Fibroblasten oder anderen Kollagen synthetisierenden Zellen oder in der Organkultur von Calvarien oder anderen Kollagen produzierenden Organen nachgewiesen werden.

Die Hemmwirkung auf die $C1_q$-Biosynthese kann in der Zellkultur mit Makrophagen bestimmt werden (Müller et al., FEBS Lett., a.a.O.).

Die Verbindungen der Formel (I) können als Medikamente in Form pharmazeutischer Präparate Verwendung finden, welche sie gegebenfalls zusammen mit verträglichen pharmazeutischen Trägern enthalten. Die Verbindungen können als Heilmittel, z.B. in Form pharmazeutischer Präparate Verwendung finden, welche diese Verbindungen in Mischung mit einem für die enterale, perkutane oder parenterale Applikation geeigneten pharmazeutischen, organischen oder anorganischen Trägermaterial, wie z.B. Wasser, Gummi arabicum, Gelatine, Milchzucker, Stärke, Magnesiumstearat, Talk, pflanzliche Öle, Polyalkylenglykole, Vaseline usw., enthalten.

Die pharmazeutischen Präparate können in fester Form, z.B. als Tabletten, Dragées, Suppositorien oder Kapseln; in halbfester Form, z.B. als Salben, oder in flüssiger Form, z.B. als Lösungen, Suspensionen oder Emulsionen, vorliegen. Gegebenenfalls sind sie sterilisiert und/oder enthalten Hilfsstoffe wie Konservierungs-, Stabilisierungs-, Netz- oder Emulgiermittel, Salze zur Veränderung des osmotischen Druckes oder Puffer. Sie können auch noch andere therapeutisch wirksame Stoffe enthalten.

Verbindungen der Formel (I) können als Insektenabwehrmittel (US-A 2 852 519) oder Ausgangsmaterialien für Bakterizide (US-A 2 809 146) dienen. Ihre Herstellung kann nach literaturbekannten Methoden, beispielsweise nach dem Verfahren der US-A 2 852 519, erfolgen.

Im folgenden wird die Bestimmung der Hemmwirkung beschrieben.

a) Gewebe des EHS-Sarkom-Tumor, der eine basalmembranartige extrazelluläre Matrix und speziell Typ IV-Kollagen produziert (Erkin et al., Exp. Med. 145 [1977] 204–220), wird analog zu der Methode von K. Tryggvason et al. (Biochemistry 19 [1980] 1248–1289) in Gegenwart von $^{14}C$-Prolin und von Inhibitoren verschiedener Konzentration inkubiert. Nach Abstoppen der Inkubation wird das Gewebe homogenisiert und mit verdünnter Essigsäure extrahiert. Nach NaCl-Fällung wurde

das extrahierte Kollagen mit 6 M HCl hydrolysiert und das Verhältnis $^{14}C$-Prolin zu $^{14}C$-Hydroxyprolin bestimmt.

b) Isolierte Calvarien werden analog der Methode von B. Peterkovsky und R. DiBlasio (Anal. Biochem. 66 [1975] 279–286) in Gegenwart von U-$^{14}C$-Prolin und Inhibitoren inkubiert. Nach Stoppen der Inkubation werden die Calvarien homogenisiert und das Kollagen mit verdünnter Essigsäure extrahiert. Nach Hydrolyse des Extraktes mit 6 M HCl wird das Verhältnis von $^{14}C$-Prolin zu $^{14}C$-Hydroxyprolin bestimmt. Folgende Ergebnisse wurden erhalten:

Inhibitor der Formel (I):

| $R^1 = R^2$ | Stellung von $COOR^2$ | Konz., µM | Hemmung, % |
|---|---|---|---|
| H | 4 | 670 | 50 |
| $C_2H_5$ | 4 | 10 | 70 |
| H | 5 | 2100 | 50 |
| $C_2H_5$ | 5 | 90 | 50 |

**Patentansprüche für die Vertragsstaaten: BE, CH, DE, FR, GB, IT, LI, LU, NL, SE**

1. Verbindungen der Formel (I)

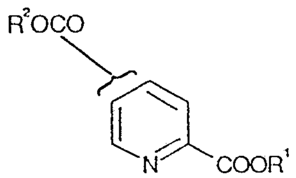

I

in der $R^1$ und $R^2$, die gleich oder verschieden sind, Alkyl mit 1 bis 6 Kohlenstoffatomen bedeuten, wobei die Gruppe $R^2OCO$ in 4- oder 5-Stellung des Pyridinrings gebunden ist, zur Anwendung als Arzneimittel.

2. Verbindungen nach Anspruch 1, in denen $R^1$ und $R^2$ für Alkylreste mit 1 bis 3 Kohlenstoffatomen stehen.

3. Verbindungen nach Anspruch 1 und 2, in denen $R^1$ und $R^2$ für Ethyl stehen.

4. Verbindungen nach Anspruch 1 bis 3 zur Inhibierung der Prolin- und Lysinhydroxylase bzw. zur Anwendung als Fibrosuppressiva und Immunsuppressiva.

5. Arzneimittel, bestehend aus einer Verbindung der Formel (I) bzw. enthaltend eine Verbindung der Formel (I).

6. Arzneimittel, enthaltend eine Verbindung der Formel (I) mit verträglichen pharmazeutischen Trägern.

7. Verwendung von Verbindungen der Formel (I) zur Herstellung eines Arzneimittels für die Beeinflussung des Stoffwechsels von Kollagen und kollagenähnlichen Stoffen bzw. der Biosynthese von $C1_q$.

8. Verwendung von Verbindungen der Formel (I) zur Herstellung eines Arzneimittels für die Behandlung von Störungen des Stoffwechsels von Kollagen und kollagenähnlichen Stoffen bzw. der Biosynthese von $C1_q$.

9. Verfahren zur Herstellung von Arzneimitteln zur Beeinflussung des Stoffwechsels von Kollagen und kollagenähnlichen Stoffen bzw. der Biosynthese von $C1_q$, dadurch gekennzeichnet, dass man in das Arzneimittel eine Verbindung der Formel (I) einverleibt.

**Patentansprüche für den Vertragsstaat AT**

1. Verfahren zur Herstellung von Arzneimitteln zur Beeinflussung des Stoffwechsels von Kollagen und kollagenähnlichen Stoffen bzw. der Biosynthese von $C1_q$, dadurch gekennzeichnet, dass man in das Arzneimittel eine Verbindung der Formel (I)

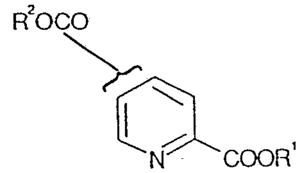

I

in der $R^1$ und $R^2$, die gleich oder verschieden sind, Alkyl mit 1 bis 6 Kohlenstoffatomen bedeuten, wobei die Gruppe $R^2OCO$ in 4- und 5-Stellung des Pyridinrings gebunden ist, einverleibt.

2. Verwendung von Verbindungen der Formel (I) zur Herstellung eines Arzneimittels für die Beeinflussung des Stoffwechsels von Kollagen und kollagenähnlichen Stoffen bzw. der Biosynthese von $C1_q$.

3. Verwendung von Verbindungen der Formel (I) zur Herstellung eines Arzneimittels für die Behandlung von Störungen des Stoffwechsels von Kollagen und kollagenähnlichen Stoffen bzw. der Biosynthese von $C1_q$.

**Claims for the Contracting States BE, CH, DE, FR, GB, IT, LI, LU, NL, SE**

1. A compound of the formula (1)

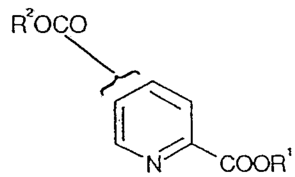

I

in which $R^1$ and $R^2$, which are identical or different, denote alkyl having 1 to 6 carbon atoms, the group $R^2OCO$ being bonded in the 4- or 5-position of the pyridine ring, for use as a medicament.

2. A compound as claimed in claim 1, in which $R^1$ and $R^2$ represent alkyl radicals having 1 to 3 carbon atoms.

3. A compound as claimed in claim 1 and 2, in which $R^1$ and $R^2$ represent ethyl.

4. A compound as claimed in claim 1 to 3 for inhibition of proline hydroxylase and lysine hydroxylase and for use as a fibrosuppressant and immunosuppressant.

5. A medicament composed of a compound

of the formula (I) or containing a compound of the formula (I).

6. A medicament containing a compound of the formula (I) with tolerated pharmaceutical vehicles.

7. The use of compounds of the formula (I) for the preparation of a medicament for affecting the metabolism of collagen and collagen-like substances and the biosynthesis of $C1_q$.

8. The use of compounds of the formula (I) for the preparation of a medicament for the treatment of disturbances of the metabolism of collagen and collagen-like substances and of the biosynthesis of $C1_q$.

9. A process for the preparation of medicaments for affecting the metabolism of collagen and collagen-like substances and the biosynthesis of $C1_q$, which comprises incorporation of a compound of the formula (I) in the medicament.

**Claims for the contracting state AT**

1. A process for the preparation of medicaments for affecting the metabolism of collagen and collagen-like substances and the biosynthesis of $C1_q$, which comprises incorporation in the medicament of a compound of the formula (I)

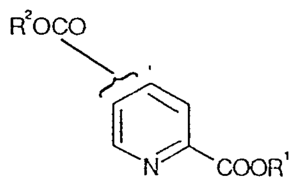

I

in which $R^1$ and $R^2$, which are identical or different, denote alkyl having 1 to 6 carbon atoms, the group $R^2OCO$ being bonded in the 4- or 5-position of the pyridine ring.

2. The use of compounds of the formula (I) for the preparation of a medicament for affecting the metabolism of collagen and collagen-like substances and the biosynthesis of $C1_q$.

3. The use of compounds of the formula (I) for the preparation of a medicament for the treatment of disturbances of the metabolism of collagen and collagen-like substances and of the biosynthesis of $C1_q$.

**Revendications pour les Etats contractants BE, CH, DE, FR, GB, IT, LI, LU, NL, SE**

1. Composés de formule (I)

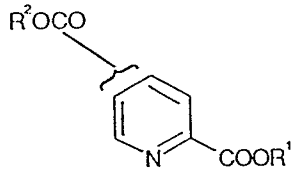

I

dans laquelle $R^1$ et $R^2$, qui sont identiques ou différents, représentent un radical alkyle à 1 à 6 atomes de carbone, le groupe $R^2OCO$ étant fixé en position 4 ou 5 du cycle pyridinique, destinés à être utilisés comme médicaments.

2. Composés selon la revendication 1 dans lesquels $R^1$ et $R^2$ représentent des radicaux alkyle à 1 à 3 atomes de carbone.

3. Composés selon les revendications 1 et 2 dans lesquels $R^1$ et $R^2$ représentent le radical éthyle.

4. Composés selon les revendications 1 à 3 destinés à l'inhibition de la proline- et lysinehydroxylase ou à l'utilisation comme fibrosuppresseurs et immunosuppresseurs.

5. Médicaments constitués d'un composé de formule (I) ou contenant un composé de formule (I).

6. Médicaments contenant un composé de formule (I) avec des véhicules pharmaceutiquement acceptables.

7. Utilisation de composés de formule (I) pour la fabrication d'un médicament destiné à agir sur le métabolisme du collagène et des substance collagéniques ou sur la biosynthèse de $C1_q$.

8. Emploi de composés de formule (I) pour la fabrication d'un médicament pour le traitement des troubles du métabolisme du collagène et des substances collagéniques ou de la biosynthèse de $C1_q$.

9. Procédé de fabrication de médicaments destinés à agir sur le métabolisme du collagène et des substance collagéniques ou sur la biosynthèse de $C1_q$, caractérisé en ce que l'on incorpore au médicament un composé de formule (I).

**Revendications pour l'Etat Contractant AT**

1. Procédé de fabrication de médicaments destinés à agir sur le métabolisme du collagène et des substances collagéniques ou sur la biosynthèse de $C1_q$, caractérisé en ce que l'on incorpore au médicament un composé de formule (I)

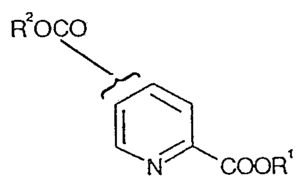

I

dans laquelle $R^1$ et $R^2$, qui sont identiques ou différents, représentent un radical alkyle à 1 à 6 atomes de carbone, le groupe $R^2OCO$ étant fixé en position 4 ou 5 du cycle pyridinique.

2. Utilisation de composés de formule (I) pour la fabrication d'un médicament destiné à agir sur le métabolisme du collagène et des substances collagéniques ou sur la biosynthèse de $C1_q$.

3. Utilisation de composés de formule (I) pour la fabrication d'un médicament pour le traitement des troubles du métabolisme du collagène et des substances collagéniques ou de la biosynthèse de $C1_q$.